Europäisches Patentamt

European Patent Office

Office européen des brevets

(19)

(11) Numéro de publication: **0 252 838**
**A1**

## (12) DEMANDE DE BREVET EUROPEEN

(21) Numéro de dépôt: 87401586.0

(22) Date de dépôt: 07.07.87

(51) Int. Cl.⁴: **A 61 K 39/10**

(30) Priorité: 08.07.86 FR 8609900

(43) Date de publication de la demande:
13.01.88 Bulletin 88/02

(84) Etats contractants désignés:
AT BE CH DE ES FR GB GR IT LI LU NL SE

(71) Demandeur: INSTITUT PASTEUR Fondation reconnue
d'utilité publique
28 rue du Docteur Roux
F-75724 Paris Cédex 15 (FR)

(72) Inventeur: Bellalou, Jacques
10 rue Gagnée
F-94400 Vitry S/Seine (FR)

(74) Mandataire: Ores, Irène et al
CABINET ORES 6, Avenue de Messine
F-75008 Paris (FR)

(54) Procédé de préparation de fractions antigéniques exocellulaires de Bordetella pertussis et vaccin contre la coqueluche contenant de telles fractions.

(57) La présente invention est relative à un procédé de préparation de fractions antigéniques exocellulaires de Bordetella pertussis.

Le procédé comprend : - une première étape au cours de laquelle une souche de B. pertussis est cultivée dans un milieu de culture approprié propre à provoquer la sécrétion des fractions antigéniques recherchées; - une deuxième étape de traitement des surnageants collectés; - et enfin une troisième étape d'extraction et de purification.

Application à la préparation de vaccin contre la coqueluche.

EP 0 252 838 A1

## Description

### PROCEDE DE PREPARATION DE FRACTIONS ANTIGENIQUES EXOCELLULAIRES DE BORDETELLA PERTUSSIS ET VACCIN CONTRE LA COQUELUCHE CONTENANT DE TELLES FRACTIONS

La présente invention est relative à un procédé de préparation de fractions exocellulaires de Bordetella pertussis, ainsi qu'à un vaccin contre la coqueluche contenant de telles fractions.

Comme on le sait, la coqueluche est une infection des voies respiratoires supérieures qui demeure encore aujourd'hui suffisamment préoccupante en France, et dans le monde, pour justifier l'incitation à la vaccination des jeunes enfants, seul moyen actuel d'en maîtriser l'incidence.

L'agent causal de la maladie est le bacille de Bordet et Gengou, Bordetella pertussis, transmis par voie aérogène d'un sujet malade à un sujet non immunisé.

Après une phase d'incubation silencieuse (2 semaines environ) et une phase catarrhale pré-quinteuse durant laquelle B. pertussis est isolable du naso-pharynx des malades, la coqueluche se manifeste essentiellement par un syndrome de toux paroxystique sans fièvre. La maladie est particulièrement grave dans les premiers mois du nourisson par l'existence de quintes asphyxiantes et d'apnées syncopales.

Les vaccins bactériens constituent les seuls moyens d'immunisation préventive dont l'efficacité est prouvée. Cependant l'existence de réactions post-vaccinales locales ou générales et l'apparition de complications neurologiques graves consécutives à une vaccination chez des enfants, dont le lien de causalité avec la vaccination n'a cependant pas été démontré, ont conduit les autorités sanitaires de certains pays à remettre en question l'opportunité de la vaccination anti-coquelucheuse. L'amélioration de l'immunogénicité des vaccins bactériens s'avère donc comme étant une nécessité.

Des progrès ont été réalisés ces dernières années dans l'isolement et la caractérisation des déterminants de pathogénicité et d'antigénicité de B. pertussis, savoir la toxine pertussis (P.T.), l'hémagglutinine filamenteuse (FHA), des agglutinogènes, la toxine dermonécrotique (TDN ou HLT), l'endotoxine (LPS), la cytotoxine trachéale (TCT), l'adényl cyclase (CYA). L'intérêt a été récemment axé sur la toxine pertussis (P.T.) et sur l'hémagglutinine filamenteuse (FHA) en raison de leur rôle dans la pathogénie de la maladie. Ils sont considérés aujourd'hui comme les deux principaux immunogènes utilisables pour la vaccination.

La toxine pertussis a fait l'objet d'études intensives car, outre sa capacité de provoquer un hyperlymphocytose (effet LPF), elle est responsable d'une sensibilité accrue à l'histamine et à la sérotonine (effet HSF), et à l'insuline (effet I.A.P.). En effet, elle est responsable de l'ADP ribosylation de la sous-unité inhibitrice de l'adénylcyclase eucaryote, provoquant une élévation du taux d'AMP cyclique intracellulaire et une amplification de la réponse enzymatique à divers stimuli hormonaux. Cette toxine est de plus un puissant mitogène à l'égard des cellules T et B et intervient dans la production accrue d'anticorps réaginiques et hémagglutinants. Il a été démontré qu'il s'agit d'une protéine de la membrane externe des bactéries, de poids moléculaire de l'ordre de 77000 à 108000 d et de structure analogue à celle des toxines diphtérique et cholérique; les toxines bactériennes de ce type sont constituées de 2 sous-unités fonctionnelles (modèles A-B). Le composant A est la sous-unité active, alors que le composant B est nécessaire à la formation du complexe toxine-cellule et à la pénétration de la sous-unité A dans la cellule. Le traitement par une solution concentrée d'Urée conduit à une séparation de la toxine en 5 peptides dont 2 sont de structure identique. Il a été démontré que seule la toxine complète est active.

L'hémagglutinine filamenteuse (FHA) serait le second déterminant immunogène d'une préparation vaccinante soluble. Supposée jouer un rôle important dans le processus de fixation des bactéries sur les cellules cibles du tissu épithélial, elle a été récemment purifiée et en grande partie caractérisée. Il s'agit d'une protéine de poids moléculaire d'environ 130000 d, capable d'assurer l'immunisation de souris infectées par des bactéries injectées sous forme d'aérosol, mais inefficace contre une infection intracérébrale.

B. pertussis est une bactérie aérobie, auxotrophe, qui a été utilisée pour la première fois en 1906 par Bordet et Gengou sur un milieu gélosé à base d'extrait de pomme de terre additionné de sang frais encore utilisé actuellement pour l'identification en routine de B. pertussis , la conservation des souches et la préparation de lots de suspensions bactériennes lyophilisées. Le milieu de Bordet et Gengou (Milieu BG) assure en général l'isolement de souches en phase I qui est le phénotype possédant les propriétés de synthèse de toxines et de divers immunogènes.

Divers milieux de culture ont été proposés dans l'optique d'une fabrication industrielle de vaccins anticoquelucheux cellulaires, en visant à une production importante de biomasse et à une immunogénicité élevée des suspensions bactériennes.

Les milieux de culture les plus largement utilisés sont le milieu de Cohen et Wheeler (Amer. J. Publ. Hlth., 1946, 36, 371) et le milieu de Stainer et Scholte (J. Gen. Microbiol., 1970, 63, 211-220.

Dans certaines conditions de culture, B. pertussis libère dans le milieu des métabolites tels que la P.T., la FHA et l'adénylcyclase. La présence de P.T. et de FHA dans les surnageants de culture permet d'envisager la mise au point d'un vaccin acellulaire dépourvu des effets indésirables observés chez l'enfant après injection de vaccin constitué par des bactéries entières inactivées.

C'est ainsi que EP 47802 a proposé une méthode de production de P.T. par isolement de cette dernière à partir d'un surnageant de culture d'une souche de B. pertussis phase I (Tohama), - ou d'un concentré de ce surnageant obtenu par relargage par du sulfate d'ammonium -, par centrifugation à 62000 - 122000 g sur un gradient de densité de saccharose (ou de tartrate de potassium ou de chlorure de césium) entre 0 et 60% Poids/Poids pendant 10-24 heures environ, cet isolement étant suivi de la floculation de la P.T. par addition de formaldéhyde, en l'absence d'amino-acides, pour détoxifier la P.T.

Cependant, compte tenu de l'importance immunogénique probable de la FHA, une composition vaccinante efficace devrait contenir à la fois de la P.T. et de la FHA. Or la culture en milieu liquide statique utilisée pour la production de FHA exocellulaire procure des rendements relativement faibles, auxquels s'ajoutent des difficultés de mise en oeuvre industrielle, alors que les surnageants de cultures agitées ne permettent de retrouver que de la P.T. er fin de culture (48-60 heures) car la FHA est détruite relativement tôt (16 heures maximum de culture) par l'agitation importante des cultures. C'est la raison pour laquelle il a été proposé récemment (EP 77646) d'utiliser un milieu de culture de B. pertussis de Stainer et Scholte qui contient cependant en outre une cyclodextrine (notamment une $\beta$-cyclodextrine méthylée) qui a pour propriété de stimuler les synthèses antigéniques et de préserver leurs activités biologiques respectives des dégradations habituellement observées en cours de culture (notamment dues à l'agitation des cultures). Les résultats qui figurent dans cette demande de brevet européen déposée au nom de TEIJIN Limited ne font cependant pas apparaître l'existence dans la préparation vaccinante obtenue à partir du surnageant, d'un autre immonogène que la P.T.. EP 121 249 également au nom de TEIJIN, propose de contrôler la quantité d'oxygène dissous (la DO), les conditions de démoussage, ainsi que la température et le pH du milieu de culture, pour augmenter la production des deux immunogènes recherchés (P.T. et FHA).

Comme on le sait, le milieu de Stainer et Scholte (Référence citée) présente la composition suivante :

|  | g/litre |
|---|---|
| L-proline | 0,242 |
| Glutamate de sodium | 10,71 |
| NaCl | 2,5 |
| $KH_2PO_4$ | 0,5 |
| KCl | 0'2 |
| $MgCl_2 \cdot 6H_2O$ | 0,1 |
| $CaCl_2$ | 0,02 |
| Tampon Tris | 6,07 |
| L-cystine | 0,04 |
| Glutathion | 0,1 |
| Acide ascorbique | 0,02 |
| Niacine | 0,004 |
| $FeSO_4 \cdot 7H_2O$ | 0,01 |

Il s'est avéré que le tampon Tris présent dans le milieu de culture de Stainer et Scholte affecte l'organisation structurale de la membrane externe des cellules Gram-négatives en se fixant aux structures de surface de cette dernière telles que lipopolysaccharides (LPS) qu'il contribuerait à entraîner. Par suite, LOTHE et Al. (Journal of Biological Standardization, 1985, 13, 129-134) ont proposé de modifier le milieu de Stainer et Scholte en remplaçant le Tampon Tris par du glycérophosphate de sodium, présent à raison de 2,5 g/l, alors que pour éliminer cet inconvénient du Tris, STAINER et SCHOLTE en avaient déjà diminué la teneur dans leur milieu de culture, jusqu'à 1,520 g/l, comme indiqué dans la publication de LOTHE et Al.

Comme mentionné plus haut, selon EP 47802, la P.T. est isolée d'un surnageant de culture par centrifugation sur un gradient de densité de saccharose ou de tartrate de potassium ou de chlorure de césium ou par gel-filtration.

Selon EP 77646 la P.T. est isolée du surnageant de culture en faisant passer celui-ci sur une première colonne d'hydroxyapatite équilibrée avec un tampon phosphate 0,01 M(pH 8,0), la solution résultante après ajustement à pH6 étant passée sur une deuxième colonne d'hydroxyapatite équilibrée avec un tampon phosphate 0,01 M (pH 6,0) et la protéine fixée est éluée par du tampon phosphate 0,1 M (pH 7,0) contenant du

3

CINa 0,5 M, l'éluat étant lui-même traité par chromatographie d'affinité pour recueillir la P.T. et la FHA respectivement.

Selon EP 121249, le précipité obtenu par addition de sulfate d'ammonium au surnageant de culture résultant d'une culture agitée, et recueilli par centrifugation ou filtration, est dissous dans un tampon phosphate (pH 7,2) contenant 1 mole de CINa; cette solution est à nouveau précipitée par du sulfate d'ammonium et le précipité séparé par centrifugation ou filtration est dialysé contre le tampon phosphate susdit. La solution résultante est ultracentrifugée et le surnageant est soumis à une centrifugation sur un gradient de densité de saccharose. La totalité du processus est menée à une température qui est de préférence égale ou inférieure à 4°C. Les fractions antigéniques obtenues contiennent une quantité importante de P.T. et de FHA.

EP 162639 propose de traiter une culture de cellules de B. pertussis par un tampon à pH 2,5-3,5 contenant de l'acide chlorhydrique et des acides aminés (glycine ou alanine) pour isoler du surnageant résultant, une préparation antigénique contenant une matière protéique présentant de l'activité adénylatecyclase (ACAP) qui est purifiée par chromatographie par échange d'ions et/ou précipitation isoélectrique, éventuellement suivis d'une immunosorption.

Il a également été proposé de concentrer des extraits cellulaires provenant de cultures agitées de B. pertussis, par ultrafiltration sous vide, pour obtenir une fraction riche en P.T. et dépourvue de FHA détectable, l'ultrafiltration constituant la dernière étape d'un traitement des extraits cellulaires comprenant des étapes successives de dialyse avec formation d'un floculat, de centrifugation du floculat, d'extraction du précipité obtenu, puis de chromatographie de l'extrait recueilli (MUNOZ et Al. INF. IMMUN. 32, 1981, 243-250).

La présente invention s'est donné pour but de pourvoir à la production de surnageants de culture de teneur élevée en immunogènes de B. pertussis (P.T. et FHA) à partir desquels sont isolés et purifiés ces immunogènes pour la préparation d'un vaccin anti-coquelucheux acellulaire de complète innocuité.

La présente invention a pour objet un procédé de préparation de fractions antigéniques exocellulaires de Bordetella pertussis obtenues par traitement de surnageants de cultures de B. pertussis, qui est caractérisé en ce qu'il comprend : - une première étape au cours de laquelle une souche appropriée de B. pertussis présentant les caractères culturaux des souches en phase I, est cultivée dans un milieu de culture approprié propre à provoquer la formation d'une densité bactérienne élevée et la sécrétion des fractions antigéniques recherchées, essentiellement P.T. et FHA, dans le surnageant de culture, à stimuler les synthèses antigéniques et à préserver les fractions antigéniques excrétées, des dégradations qu'elles sont susceptibles de subir en cours de culture, ladite culture étant réalisée en pourvoyant à un apport d'oxygène par injection d'air et/ou d'oxygène en profondeur, sous une pression d'oxygène dissous régulée à 40% de saturation, dans des conditions d'agitation modérée, de l'ordre de 100 tours/minute, en présence de moyens de destruction de la mousse formée sous l'effet de l'agitation, pendant une durée de 44 à 48 heures; - une deuxième étape de traitement des surnageants collectés en fin de culture, c'est-à-dire à l'épuisement des substrats azotés, qui consiste à séparer de surnageant de culture des cellules bactériennes, par sédimentation par centrifugation, à ajuster le pH du surnageant re cueilli, à pH 3-4, par addition d'acide chlorhydrique concentré, et à le laisser reposer à 4°C environ, pendant 3 semaines environ au bout desquelles le culot obtenu au cours de cette phase de décantation et constitué par des bactéries, des protéines du milieu et des fractions antigéniques, est récupéré par centrifugation et extrait en tampon pyrophosphate 0,1 M, NaCl 0,5 M, pH 7-7,2, sous agitation douce, puis le produit de l'extraction est soumis à une seconde centrifugation suivie d'une nouvelle extraction par le même tampon, pour récupérer des extraits clairs présentant des activités P.T. et FHA élevées, qui sont isolées par concentration ou purification par chromatographie.

Selon un mode de mise en oeuvre préféré du procédé qui fait l'objet de la présente invention, la souche de B. pertussis mise en culture est constituée par un inoculum constitué par des suspensions bactériennes cultivées pendant 24 heures à 36°C sur du milieu BG en boîtes de Roux, lequel inoculum est avantageusement ensemencé en quantité de l'ordre de 1 U10/ml.

Selon un autre mode de mise en oeuvre préféré du procédé qui fait l'objet de la présente invention, le milieu de culture de la souche de B. pertussis est avantageusement un milieu de Stainer et Scholte modifié contenant une cyclodextrine et une quantité relativement faible de tampon Tris (environ 1,25 g/litre).

Ce milieu est décrit dans la demande de brevet EP 121249.

Selon encore un autre mode de mise en oeuvre préféré du procédé qui fait l'objet de la présente invention, l'apport d'oxygène est assuré par injection d'air en profondeur à raison de 0,71 VVM.

Selon une autre disposition de l'invention, l'apport d'oxygène est assuré par injection d'oxygène en profondeur à raison de 0,14 VVM, l'injection d'air et d'oxygène pouvant être combinée à raison d'un débit d'aération de 0,28 VVM.

Conformément à l'invention, les moyens de destruction de la mousse sont des moyens mécaniques.

Selon une autre disposition de l'invention, les moyens de destruction de la mousse sont constitués par un agent anti-mousse avantageusement ajouté au milieu de culture en quantité pouvant atteindre 7,5 ml/litre de milieu.

Selon un mode de mise en oeuvre préféré du procédé conforme à la présente invention, l'apport d'oxygène en profondeur à la biomasse est tel que la concentration en oxygène dissous soit de 2,8 mg/l à 37°C, que la demande en oxygène qui peut atteindre 6 mmoles $O_2$ /l/h en fin de phase exponentielle de croissance bactérienne, soit satisfaite, que le transfert d'oxygène soit d'environ 12 mmoles d'$O_2$ /l/h, que la consommation spécifique d'oxygène soit de l'ordre de 3,54 g $O_2$ /g de biomasse - ou 9,96 mmoles $O_2$ /mole de biomasse - assurant ainsi une croissance bactérienne optimale, avec un rendement massique de conversion du substrat en biomasse de l'ordre de 0,18-0,20 g/g.

Selon une variante de réalisation du procédé conforme à la présente invention, les surnageants collectés en fin de culture sont soumis à une ultrafiltration qui réalise à la fois la filtration et la concentration rapides du surnageant, puis à un traitement de récupération des fractions antigéniques P.T. et FHA par tous moyens appropriés, et notamment par chromatographies sur colonnes.

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de préparation des fractions antigéniques et de caractérisation de ces dernières.

Il doit être bien entendu, toutefois, que ces exemples de mise en oeuvre, sont donnés uniquement à titre d'illustration de l'objet de l'invention, dont ils ne constituent en aucune manière une limitation.

## EXEMPLE 1

### 1. Culture de B. pertussis

L'évolution des activités LPF et HA étant couplée à la multiplication bactérienne, des conditions de culture assurant une croissance rapide ne s'arrêtant qu'à l'épuisement des substrats azotés (acide glutamique et casaminoacides) ont été recherchées. Compte-tenu de la sensibilité du FHA aux contraintes de cisaillement, une vitesse d'agitation modérée (100 tr/min) a été adoptée. L'apport d'oxygène est assuré par injection d'air (0,71 VVM) ou d'oxygène (0,14 et 0,28 VVM), en profondeur à travers un tamis microperforé, habituellement utilisé en culture cellulaire.

Une pression d'oxygène dissous, régulée à 40% de saturation a été choisie, à la suite de la détermination des capacités de transfert du réacteur de 10 litres utilisé, soumis à une aération en profondeur dans les mêmes conditions expérimentales (agitation de 100 tr/min) (détermination du $K_L.a$ ou coefficient volumétrique de transfert d'oxygène exprimé en $h^{-1}$).

L'augmentation progressive de la quantité d'antimousse ajoutée au milieu de culture (jusqu'à 7,5 ml/litre de milieu), a permis de réduire la formation de mousse et par conséquent, d'obtenir une croissance bactérienne satisfaisante. Dans ces conditions, la croissance est exponentielle pendant 24 heures de culture. Le taux de croissance maximum est alors de 0,094 $h^{-1}$, ce qui correspond à un temps de doublement minimum de 3,2 heures. La densité bactérienne en fin de culture est de 140 U10/ml, soit 2,80 g/l en M.S. Cette valeur n'a jamais pu être atteinte en culture aérée par injection d'air en surface (1,60 g/l maximum) démontrant une limitation de l'apport d'oxygène par ce type d'aération.

L'activité du HA du milieu augmente régulièrement. L'activité LPF est la plus élevée à 25 heures de culture, puis chute légèrement en phase stationnaire. La toxine pertussis est probablement sensible à la formation de mousse. Bien que la dose d'antimousse utilisée ne semble pas inhiber la croissance et les synthèses antigéniques, une destruction mécanique de la mousse pourrait lui être préférée.

Les résultats obtenus précédemment montrent qu'une augmentation de la production d'antigènes P.T. et FHA est associée à une croissance bactérienne rapide. La vitesse du transfert d'oxygène permet de moduler cette croissance. Il est important, pour extrapoler les résultats décrits en fermenteur de 10 litres, à des réacteurs de volume supérieur (50 à 100 litres), de déterminer entre autres, le besoin en oxygène des bactéries. Celui-ci est défini par le coefficient $QO_2$ qui exprime la quantité d'oxygène consommé exprimée en volume par unité de biomasse (mmole $O_2$ /g de M.S./h). La méthode dynamique de Taguchi et Humphrey a été utilisée. Dans un premier temps, l'équilibre entre le transfert d'oxygène et la consommation bactérienne est réalisé. La concentration en $O_2$ dissous de la culture est constante et la vitesse de transfert $dc_L/dt$ est nulle :

$$\frac{dc_L}{dt} = 0 = \underbrace{K_L.a \left[ c^x - c_L \right]}_{\text{transfert d'O}_2} - \underbrace{Q_{O_2} X}_{\text{consommation bactérienne}}$$

avec $c_L$ : concentration en oxygène dissous dans la phase liquide mg/l ou mmole $O_2$ /l
$c^*$ : concentration saturante en oxygène dissous à la température de l'expérience; c = 7 mg/l à 37°C
$K_L.a$ : cofficient volumétrique de transfert d'oxygène ($h^{-1}$)
$Q_{O_2}$ : consommation cellulaire d'oxygène par unité de M.S. de biomasse µl/h.g
X : concentration en biomasse g/l de M.S.

Dans un deuxième temps, l'aération est stoppée. La concentration en oxygène dissous, $c_L$, diminue linéairement en fonction du temps, du fait de la consommation bactérienne qui se poursuit

$$\frac{d_{c_L}}{d_t} = - Q_{O_2} \cdot X$$

La pente de la droite obtenue est égale à $Q_{O_2} \cdot X$. De cette valeur est déduit le besoin en oxygène des bactéries $Q_{O_2}$.

Au taux d'aération de 0,28 VVM et pour une agitation de 100 tr/min, la valeur du besoin en oxygène déterminée pour plusieurs essais de culture, est de 4,7 à 5 mmoles $O_2$ /g/h. La demande en oxygène maximale en fin de phase exponentielle est de l'ordre de 6 mmoles $O_2$ /l/h. Les conditions expérimentales d'aération et d'agitation adoptées, assurent un transfert d'oxygène d'environ 12 mmoles $O_2$ /l/h et donc une croissance dans des conditions non limitantes.

2. Isolement et récupération des fractions antigéniques du surnageant

Après sédimentation des cellules bactériennes par centrifugation continue (rotor TZ-28 sur centrifugeuse SERVAL RC2B), le milieu de culture est ajusté à pH 3,8 à 4, par addition d'acide chlorhydrique concentré, puis stocké durant 3 semaines environ à 4°C. Des durées plus courtes de décantation ont été testées, mais les rendements d'isolement de la P.T. sont alors beaucoup plus faibles. Le surnageant est siphoné et le culot constitué de bactéries, de protéines du milieu et de fractions antigéniques, est récupéré par centrifugation (13000 g - 20 min à 4°C). Le précipité protéique est ex trait en tampon pyrophosphate 0,1 M, NaCl 0,5 M, pH 7-7,2, sous agitation magnétique douce, durant une nuit (15 heures à 4°C). Une seconde centrifugation puis une nouvelle extraction par le même tampon, permettent de récupérer un pool d'extraits clairs et d'activités LPF et HA très élevées. Des volumes de surnageants de 50 à 200 litres ont ainsi été traités. Le Tableau 1 ci-après regroupe les résutats obtenus.

0 252 838

Afin de contrôler l'efficacité de ce type de préparation, en termes de pouvoir protecteur, un échantillon dilué (0,5 ml du pool P$_3$ dilué au 1/40) a été adsorbé sur gel de phosphate de calcium et injecté à des souris

Tableau **1** : Taux de protéine et activités LPF et HA des fractions antigéniques isolées à partir de surnageants de culture en fermenteur de 50 litres, par précipitation à l'acide chlorhydrique.

| Pool | Milieu de culture et souche | Temps de culture heures | Protéine de l'extrait µg/ml | Toxine pertussis | | Activité LPF | | | Activité FHA | |
|------|------|------|------|------|------|------|------|------|------|------|
| | | | | µg/ml ELISA [1] | % de l'extrait | Dose de protéines injectées | Titre in vivo | Activité spécifique [2] | dilution | Activité spécifique [3] |
| P$_2$ | MCW.S.509 | 30 | 1000 | 460 | 46 | 12,5 | 12,9 | 1,03 | 1/256 | 0,4 |
| P$_3$ | MCW.S.509 | 60 | 3875 | 1640 | 42,3 | 12,1 | 11,3 | 0,94 | 1/64 | 6 |
| P$_4$ | MCW.S.134 | 60 | 3675 | 1500 | 41 | 11,5 | 9,6 | 0,84 | 1/1024 | 0,4 |

1 : Titre ELISA déterminé par F. Megret, Service des antigènes bactériens, I.P.F. Paris. Technique en cours de publication.

2 : Activité spécifique LPF : titre LPF / µg de protéine injectée

3 : Activité spécifique HA : plus petite quantité de protéines (µg) provoquant une réaction d'hémagglutination positive de globules rouges de mouton.

infectées par voie intracérébrale, selon le test de Kendrick. Dans ces conditions, 20 µg de toxine pertussis ont induit une protection chez la souris de 5,7 UI. De plus, il a été vérifié que l'addition d'une dose subtoxique d'une préparation de ce type à un vaccin bactérien préalablement titré et ne satisfaisant pas aux normes d'activité (titre : < 4UI/dose humaine), restitue aux bactéries leur pouvoir protecteur (Tableau 2 ci-après)

Tableau 2 : Effets de l'addition d'une dose subtoxique d'une préparation soluble de teneur élevée en toxine pertussis et en hémagglutinine filamenteuse à un vaccin bactérien

| Echantillon | Activité LPF | | Titre protecteur UI : unité internationale de protection |
| | Dose injectée µg de protéine ou UIO | Titre LPF | |
|---|---|---|---|
| Extrait soluble | 2,9 µg | 4,6 | - |
| Vaccin bactérien | 20 UIO | 2,1 | 3,2<br>1,5 - 6,6 |
| Préparation mixte | 2,9 µg+20 UIO | 9 | 4,5<br>1,7 - 12 |

EXEMPLE 2

L'étape 2 de l'Exemple 1 a été remplacée par un traitement par ultrafiltration en procédant comme suit : L'isolement de la P.T. et du FHA excrétés dans le milieu de cultures industrielles réalisées en réacteurs de volume important (200 et 500 litres), nécessite de disposer de surnageants clairs propres aux opérations de concentration ou de purification par chromatographie.

Plusieurs techniques de filtration de milieux troubles recueillis par centrifugation continue, ont été testées : filtre Seitz, filtration sur métafiltre en présence de Hyflo, filtration tangentielle sur cellule Pellikon (système Millipore). Les critères d'évaluation du procédé ont été la capacité de filtration et la nonrétention des antigènes par l'élément filtrant. Les meilleurs résultats ont été obtenus sur système Pellikon. Les filtres en amiante Seitz et l'Hyflo provoquent l'adsorption des fractions antigéniques solubles, surtout du FHA.

Dans le cas des surnageants de cultures en milieu complexe, pour lesquelles l'amidon provoque un colmatage rapide des cassettes (cassette 0,45 µ. Réf HVLP 00065), l'addition d'amylase (2,5 mg/l. 24 h à 4°C pH 6), a permis d'améliorer nettement les capacités de filtration. La concentration de la P.T. et du FHA a ensuite été réalisée sur le même système Millipore, en utilisant une cassette de point de coupure de 10.000 (cassette Réf PTGC 0005).

Les résultats de filtration et de concentration, d'environ 100 litres de milieu, sont présentés dans le Tableau 3 ci-dessous. Le rapport de concentration volumique est d'environ 55 fois.

**Tableau 3** : Concentration de la P.T. et du FHA par ultrafiltration de surnageants de cultures sur cellule Pellikon

| Etape du procédé | Teneur en protéine mg/ml | Activité LPF | | | Activité HA | |
|---|---|---|---|---|---|---|
| | | Dose injectée µg | Titre LPF | Activité spécifique[1] | Dilution | Activité spécifique[2] |
| Surnageant brut centrifugé | 5,9 | 2950 | 9 | 0,0031 | 1/4 | 147,5 |
| Surnageant + amylase filtré sur cassette 0,45 u | 5,7 | 2850 | 8,9 | 0,0031 | 1/4 | 142 |
| Fraction concentrée Cassette PC : 10 000 | 14 | 177 | 12 | 0,070 | 1/64 | 71,8 |
| Ultrafiltrat | 5,33 | 2668 | 0 | - | - | - |

1 : Activité spécifique LPF : titre LPF / µg de protéine

2 : Activité spécifique HA : plus petite quantité de protéine (µg) provoquant une réaction positive d'hémagglutination de globules rouges de mouton.

Les fractions concentrées contiennent environ 40 à 45% de P.T. La teneur en FHA est par contre plus dépendante de la concentration initiale des surnageants en hémagglutinine filamenteuse, et donc liée à la technique de culture, plutôt qu'à la technique de concentration utilisée.

L'ultrafiltration sur cellule Pellikon présente l'avantage de permettre la filtration et la concentration rapide de volumes importants de surnageants directement en fin de culture bactérienne, et d'éviter ainsi l'étape de centrifugation en continu. Les rendements sont cependant limités par une précipitation des antigènes solubles au-delà d'un seuil de concentration (40 à 50 fois). Cette méthode est sans doute la plus adaptée à la concentration partielle des surnageants avant la purification des fractions antigéniques par chromatographie sur colonne.

EXEMPLE 3

Le milieu de culture mis en oeuvre dans l'Exemple 1, présente la composition énoncée dans le Tableau 4 ci-après :

Tableau 4 : Composition des milieux liquides[a] de culture de B. pertussis (concentration en g/l)

| Nature des composants | Milieu CL [c] |
|---|---|
| L-Glutamate de sodium | 10,7 |
| Casamino acides (Difco) | 10,0 |
| L-Proline | 0,24 |
| Chlorure de sodium | 2,5 |
| Chlorure de calcium, $7H_2O$ | 0,02 |
| Chlorure de potassium | 0,2 |
| Dihydrogénophosphate de potassium | 0,5 |
| Sulfate de Fer[b], $7H_2O$ | 0,01 |
| L-Cystéine[b] | 0,04 |
| Glutathion[b] (réduit) | 0,15 |
| Acide ascorbique[b] | 0,4 |
| Acide nicotinique[b] | 0,004 |
| Tris | 1,25 |
| pH avant stérilisation | 7,6 |

a. Composition pour un litre de milieu préparé en eau distillée

b. Solution stérilisée par filtration sur membrane Millipore 0,22 µ puis additionnée stérilement au milieu de base S.S. et CL

c. Milieu stérilisé en autoclave 15 min. à 121°C.

EXEMPLE 4

Principe :
La filtration du FHA sur différents supports s'accompagne d'une perte de l'antigène dans le filtrat par adsorption de celui-ci sur la membrane de filtration. Le but de cette expérimentation est de vérifier s'il est possible d'annuler ou de limiter ce phénomène par addition de "Triton X-100" aux préparations antigéniques avant filtration. La toxine pertussis (P.T.) peut, par contre subir différents traitements de filtration sans perte antigénique.

Mode opératoire :
4 ml d'une préparation de teneur élevée en FHA sont dilués dans 40 ml de tampon Tris 50 mM, NaCl 0,2 M, pH 8. Cette préparation a été obtenue par concentration au $ZnCl_2$ d'un surnageant de culture agitée (milieu de l'Exemple 3) en erlen.
Echantillon 1 : 20 ml de préparation diluée en FHA +

10

**0 252 838**

200 µl d'une solution de Triton X-100 à 10%
(concentration finale en Triton X-100 = 0,1%).
Echantillon 2 : 20 ml de préparation diluée en FHA pas de détergent ajouté.
Les échantillons 1 et 2 sont filtrés sur membrane filtrante Millipore 0,45µm (diamètre des filtres = 4,5 cm, Ref : HAW PO 4700).
Les filtrats 1 et 2 (F1 - F2) sont récupérés, et chaque filtre est immergé dans 10 ml de tampon Tris 50 mM additionné de Triton X-100 (100 µl d'une solution à 10%).
Ces filtres sont soumis à une agitation douce, une nuit à 4°C.

Résultats :
    L'activité HA des préparations brutes diluées et des filtrats (F1 et F2), ainsi que des solutions de lavage des filtres (L1 et L2) est déterminée par le test d'hémagglutination de globules rouges de mouton.

| Echantillon | Nature | Dernière dilu-tion positive |
|---|---|---|
| E1 | FHA dilué au 1/10° + Triton X-100 | 1/2560 |
| F1 | E1 après filtration sur Millipore | 1/1280 |
| E2 | FHA dilué au 1/10° sans détergent | 1/2560 |
| F2 | E2 après filtration Millipore | 0 |
| L1 | Solution de lavage du filtre 1 | |
| L2 | Solution de lavage du filtre 2 | |

Conclusion
    L'addition de Triton X-100 en concentration finale 0,1% à des surnageants de culture, ou à des préparations concentrées, semble limiter presque totalement l'effet d'adsorption antigénique du FHA sur la membrane filtrante Millipore. Une étude plus poussée de l'influence de la composition du tampon de dilution, du pH et peut-être de la concentration optimale en Triton X-100, devrait permettre d'éliminer complètement cette perte antigénique. De tels résultats laissent penser qu'il est possible d'utiliser le système d'ultrafiltration Pellikon (membrane Millipore) pour la concentration de la P.T. et du FHA à partir de surnageants de cultures industrielles. Ces surnageants de cultures réalisées en milieu synthétique, sans amidon, se prêtent particulièrement bien à ce type de filtration et de concentration.

11

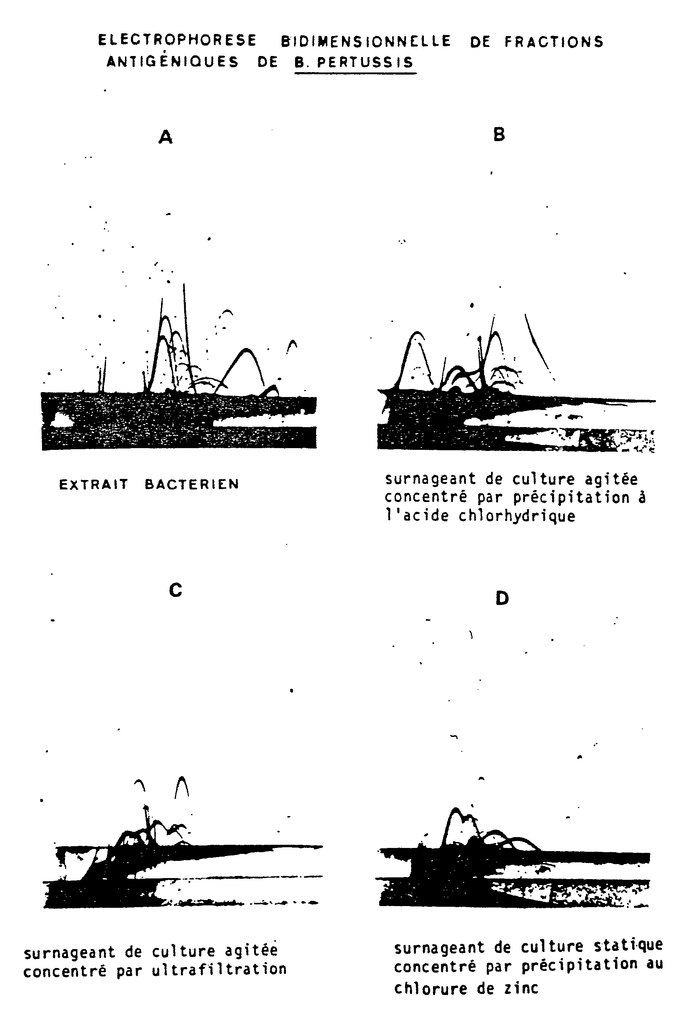

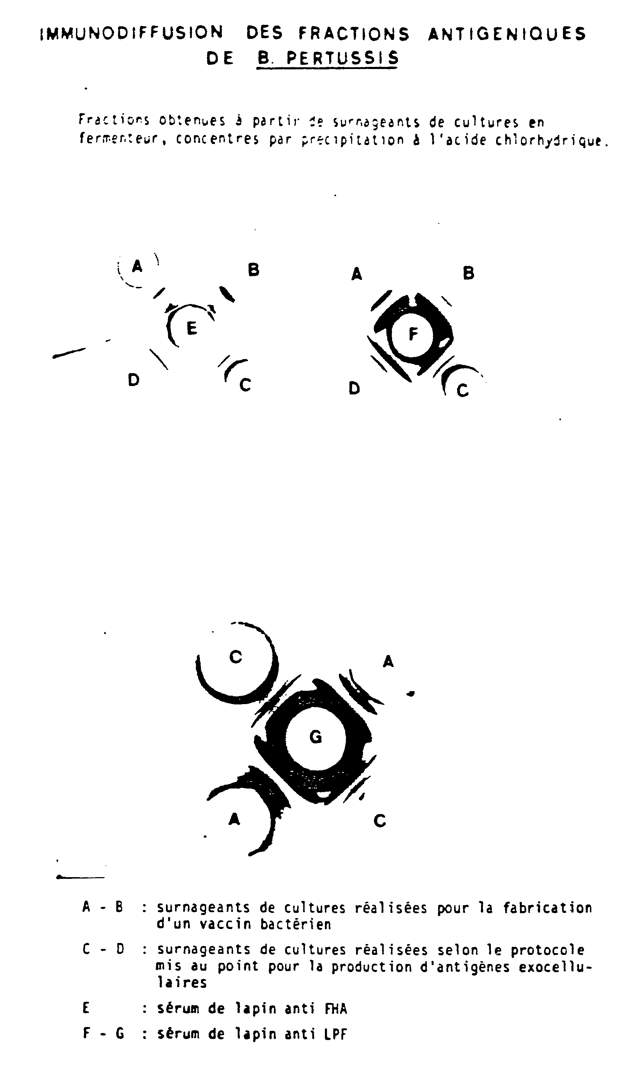

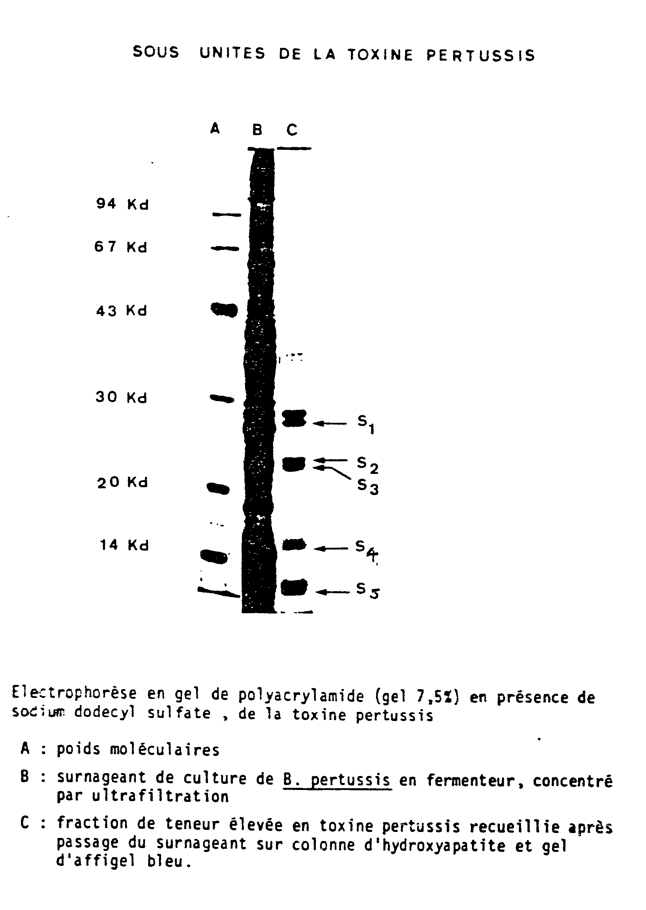

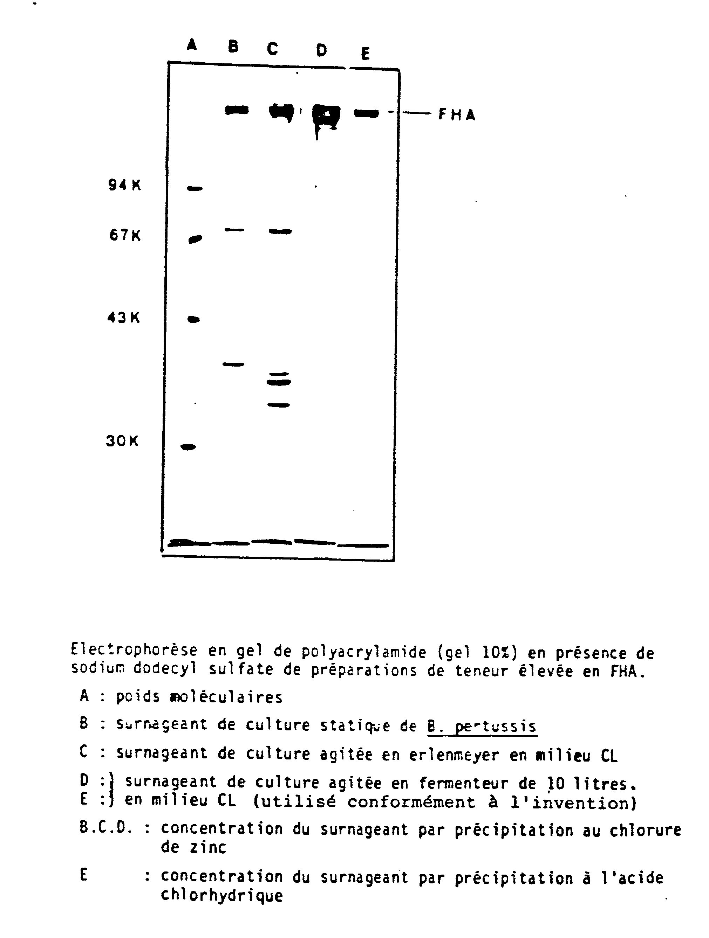

## Revendications

1°) Procédé de préparation de fractions antigéniques exocellulaires de Bordetella pertussis obtenues par traitement de surnageants de cultures de B. pertussis, caractérisé en ce qu'il comprend : - une première étape au cours de laquelle une souche appropriée de B. pertussis présentant les caractères culturaux des souches en phase I, est cultivée dans un milieu de culture approprié propre à provoquer la formation d'une densité bactérienne élevée et la sécrétion des fractions antigéniques recherchées, essentiellement P.T. et FHA, dans le surnageant de culture, à stimuler les synthèses antigéniques et à préserver les fractions antigéniques excrétées, des dégradations qu'elles sont susceptibles de subir en cours de culture, ladite culture étant réalisée en pourvoyant à un apport d'oxygène par injection d'air et/ou d'oxygène en profondeur, sous une pression d'oxygène dissous régulée à 40% de saturation, dans des conditions d'agitation modérée, de l'ordre de 100 tours/minute, en présence de moyens de destruction de la mousse formée sous l'effet de l'agitation, pendant une durée de 44 à 48 heures; - une deuxième étape de traitement des surnageants collectés en fin de culture, c'est-à-dire à l'épuisement des substrats azotés, qui consiste à séparer de surnageant de culture des cellules bactériennes, par sédimentation par centrifugation, à ajuster le pH du surnageant recueilli, à pH 3-4, par addition d'acide chlorhydrique concentré, et à le laisser reposer à 4°C environ, pendant 3 semaines environ au bout desquelles le culot obtenu au cours de cette phase de décantation et constitué par des bactéries, des protéines du milieu et des fractions antigéniques, est récupéré par centrifugation et extrait en tampon pyrophosphate 0,1 M, NaCl 0,5 M, pH 7-7,2, sous agitation douce, puis le produit de l'extraction est soumis à une seconde centrifugation suivie d'une nouvelle extraction par le même tampon, pour récupérer des extraits clairs présentant des activités P.T. et FHA élevées, qui sont isolés par con centration ou purification par chromatographie.

2°) Procédé selon la revendication 1, caractérisé en ce que la souche de B. pertussis mise en culture est constituée par un inoculum constitué par des suspensions bactériennes cultivées pendant 24 heures à 36°C sur du milieu BG en boîtes de Roux, lequel inoculum est avantageusement ensemencé en quantité de l'ordre de 1 U10/ml.

3°) Procédé selon l'une quelconque des revendications 1 et 2, caractérisé en ce que le milieu de culture de la souche de B. pertussis est avantageusement un milieu de Stainer et Scholte modifié contenant une cyclodextrine et une quantité relativement faible de tampon Tris (environ 1,25 g/litre).

4°) Procédé selon l'une quelconque des revendications 1 à 3, caractérisé en ce que l'apport d'oxygène est assuré par injection d'air en profondeur à raison de 0,71 VVM.

5°) Procédé selon l'une quelconque des revendications 1 à 3, caractérisé en ce que l'apport d'oxygène est assuré par injection d'oxygène en profondeur à raison de 0,14 VVM, l'injection d'air et d'oxygène pouvant être combinée à raison d'un débit d'aération de 0,28 VVM.

6°) Procédé selon l'une quelconque des revendications 1 à 5, caractérisé en ce que les moyens de destruction de la mousse sont des moyens mécaniques.

7°) Procédé selon l'une quelconque des revendications 1 à 5, caractérisé en ce que les moyens de destruction de la mousse sont constitués par un agent anti-mousse avantageusement ajouté au milieu de culture en quantité pouvant atteindre 7,5 ml/litre de milieu.

8°) Procédé selon l'une quelconque des revendications 1 à 7, caractérisé en ce que l'apport d'oxygène en profondeur à la biomasse est tel que la concentration en oxygène dissous soit de 2,8 mg/l à 37°C, que la demande en oxygène qui peut atteindre 6 mmoles $O_2$ /l/h en fin de phase exponen tielle de croissance bactérienne, soit satisfaite, que le transfert d'oxygène soit d'environ 12 mmoles d'$O_2$ /l/h, que la consommation spécifique d'oxygène soit de l'ordre de 3,54 g $O_2$ /g de biomasse - ou 9,96 mmoles $O_2$ /mole de biomasse - assurant ainsi une croissance bactérienne optimale, avec un rendement massique de conversion du substrat en biomasse de l'ordre de 0,18-0,20 g/g.

9°) Procédé selon l'une quelconque des revendications 1 à 8, caractérisé en ce que selon une variante du processus d'isolement des fractions antigéniques du surnageant, les surnageants collectés en fin de culture sont soumis à une ultrafiltration qui réalise à la fois la filtration et la concentration rapides du surnageant, puis à un traitement de récupération des fractions antigéniques P.T. et FHA par tous moyens appropriés, et notamment par chromatographies sur colonnes.

10°) Vaccin contre la coqueluche, caractérisé en ce qu'il est obtenu en mettant en oeuvre le procédé selon l'une quelconque des revendications 1 à 9.

11°) Vaccin contre la coqueluche, caractérisé en ce qu'il présente un pouvoir protecteur élevé, de l'ordre de 5 à 6 UI/0,5 ml, riche en P.T. (environ 20 µg/ml et possèdant un pouvoir hémagglutinant très élevé.

0252838

ELECTROPHORESE BIDIMENSIONNELLE DE FRACTIONS
ANTIGÉNIQUES DE B. PERTUSSIS

A

EXTRAIT BACTERIEN

B

surnageant de culture agitée
concentré par précipitation à
l'acide chlorhydrique

C

surnageant de culture agitée
concentré par ultrafiltration

D

surnageant de culture statique
concentré par précipitation au
chlorure de zinc

0252838

# IMMUNODIFFUSION DES FRACTIONS ANTIGENIQUES
## DE  B. PERTUSSIS

Fractions obtenues à partir de surnageants de cultures en
fermenteur, concentres par precipitation à l'acide chlorhydrique.

A - B  : surnageants de cultures réalisées pour la fabrication
         d'un vaccin bactérien

C - D  : surnageants de cultures réalisées selon le protocole
         mis au point pour la production d'antigènes exocellu-
         laires

E      : sérum de lapin anti FHA

F - G  : sérum de lapin anti LPF

0252838

## SOUS UNITES DE LA TOXINE PERTUSSIS

Electrophorèse en gel de polyacrylamide (gel 7,5%) en présence de sodium dodecyl sulfate , de la toxine pertussis

A : poids moléculaires

B : surnageant de culture de B. pertussis en fermenteur, concentré par ultrafiltration

C : fraction de teneur élevée en toxine pertussis recueillie après passage du surnageant sur colonne d'hydroxyapatite et gel d'affigel bleu.

0252838

Electrophorèse en gel de polyacrylamide (gel 10%) en présence de sodium dodecyl sulfate de préparations de teneur élevée en FHA.

A : poids moléculaires

B : surnageant de culture statique de B. pertussis

C : surnageant de culture agitée en erlenmeyer en milieu CL

D :} surnageant de culture agitée en fermenteur de 10 litres.
E :} en milieu CL (utilisé conformément à l'invention)

B.C.D. : concentration du surnageant par précipitation au chlorure de zinc

E     : concentration du surnageant par précipitation à l'acide chlorhydrique

## DOCUMENTS CONSIDERES COMME PERTINENTS

| Categorie | Citation du document avec indication, en cas de besoin, des parties pertinentes | Revendication concernee | CLASSEMENT DE LA DEMANDE (Int Cl 4) |
|---|---|---|---|
| Y,D | EP-A-0 121 249 (TEIJIN)<br>* En entier * | 1-11 | A 61 K 39/10 |
| | --- | | |
| Y | CHEMICAL ABSTRACTS, vol. 97, no. 13, 27 septembre 1982, page 336, résumé no. 115206s, Columbus, Ohio, US; H. RONNEBERGER et al.: "Pertussis antigens-screening models on toxicity", & ARCH. TOXICOL., SUPPL. 1982, 5, 129-32<br>* Abrégé * | 1-11 | |
| | --- | | |
| A | BIOLOGICAL ABSTRACTS, vol. 62, 1er octobre 1976, page 3737, résumé no. 37859, Philadelphia, US; R. BROUWER et al.: "Reactogenicity for infants of pertussis vaccines, harvested by centrifugation or acid precipitation"<br>* Abrégé * | 1 | |
| | | | **DOMAINES TECHNIQUES RECHERCHES (Int. Cl.4)**<br><br>A 61 K |
| | --- | | |
| A | NATURE, vol. 203, 15 août 1964, pages 774-775, MacMillan Journals Ltd, GB; VAN HEMERT et al.: "Preparation of soluble pertussis vaccine"<br>* Page 774, colonne 2, lignes 3-5 * | 1 | |
| | ---     -/- | | |

Le present rapport de recherche a été établi pour toutes les revendications

| Lieu de la recherche | Date d'achèvement de la recherche | Examinateur |
|---|---|---|
| LA HAYE | 23-09-1987 | SKELLY J.M. |

| Categorie | Citation du document avec indication, en cas de besoin, des parties pertinentes | Revendication concernee | CLASSEMENT DE LA DEMANDE (Int. Cl.4) |
|---|---|---|---|
| | **DOCUMENTS CONSIDERES COMME PERTINENTS** | | Page 2 |
| A | GB-A-1 145 320 (USTAV SER A OCKOVACICH LATEK) * Page 2 * | 1 | |
| A | INFECTION AND IMMUNITY, vol. 31, no. 3, mars 1981, pages 1223-1231; Y. SATO et al.: "Role of antibody to leukocytosis-promoting factor hemagglutinin and to filamentous hemagglutinin in immunity to pertussis" * En entier * | 1 | |
| A | US-A-2 405 740 (FLOSDORF) * Colonne 3, lignes 32-65; colonne 4, lignes 21-39 * | 1 | |

DOMAINES TECHNIQUES
RECHERCHES (Int. Cl.4)

Le present rapport de recherche a ete etabli pour toutes les revendications

| Lieu de la recherche | Date d'achevement de la recherche | Examinateur |
|---|---|---|
| LA HAYE | 23-09-1987 | SKELLY J.M. |